# EUROPEAN PATENT APPLICATION

(11) **EP 4 822 642 A1**
(43) Date of publication of application: **14.10.2026**
(21) Application number: 24899116.8
(22) Date of filing: 24.04.2024
(51) Int. Cl.: G06T 7/11, G06T 7/13

(54) **METHOD FOR GENERATING INTERNAL STRUCTURE OF ORGANISM ON BASIS OF DEEP LEARNING**

(30) Priority: 08.12.2023 CN 202311674971
(71) Applicant: Hefei Raycision Medical Technology Co., Ltd., Hefei, Anhui 230088 (CN)
(72) Inventor: YANG, Yirui, Hefei, Anhui 230088 (CN)
(74) Representative: M. Zardi & Co S.A.
(86) International application number: PCT/CN2024/089581
(87) International publication number: WO 2025/118464

(57) **Abstract**

The present application relates to the field of life sciences and medical imaging device technologies, and more particularly, to a deep learning-based method for generating an internal structure of an organism. The method includes the following steps: S1, constructing and training an internal organ generation model of a imaging target; S2, obtaining a three-dimensional body surface contour image of the imaging target; S3, inputting the obtained three-dimensional body surface contour image of the imaging target into the internal organ generation model to obtain a three-dimensional internal organ distribution image; and S4, performing merging on the three-dimensional internal organ distribution image and the three-dimensional body surface contour image, and displaying a result obtained based on the merging. With a deep neural network-based internal organ generation model of the imaging target, the three-dimensional internal organ distribution image of the imaging target can be obtained quickly and accurately simply through obtaining the three-dimensional body surface contour image of the imaging target and inputting the three-dimensional body surface contour image into the internal organ generation model, which simplifies operation steps, realizes automatic imaging, improves an imaging efficiency, and has high applicability.

## Description

### CROSS-REFERENCE TO RELATED APPLICATION

The present application claims priority to Chinese patent application No. 202311674971.X, titled "DEEP LEARNING-BASED METHOD FOR GENERATING INTERNAL STRUCTURE OF ORGANISM" and filed on December 8, 2023, the entire contents of which are incorporated herein by reference.

### FIELD

The present disclosure relates to the field of life sciences and medical imaging device technologies, and more particularly, to a deep learning-based method for generating an internal structure of an organism.

### BACKGROUND

Biomedical body surface imaging is a technique for image capture and analysis of a surface of an organism. Conventional body surface imaging techniques mainly include laser scanning imaging, time-of-flight imaging, stereo vision imaging, and structured light imaging.

In the laser scanning imaging, an object can be scanned using a laser beam to obtain three-dimensional morphology information of a surface of the object. Applications of the laser scanning imaging in the field of medical imaging are mainly to scan the organism using the laser beam to obtain precise image information. A basic principle of this technique is to scan the surface of the object using the laser beam and measure time and intensity information of the laser beam reflected back from the surface of the object, in such a manner that a morphology and a position of the surface of the object can be determined.

A time-of-flight imaging system includes an image sensor, an image processing chip, and a modulated light source. Simply put, an operation principle of the system is to illuminate a scene with the modulated light source and then measuring a phase difference of waves reflected back. Since a speed of light is constant, the time-of-flight imaging system can calculate a distance to each point in the scene based on the time it takes for the light to return to a camera. Instead of scanning an image line by line, the time-of-flight imaging system illuminates the entire scene sequentially and then measures a phase difference of the light reflected back to the image sensor. The time-of-flight imaging system, which can be used for large-field-of-view, long-range, low-precision, low-cost three-dimensional (3D) image collection, is characterized by fast detection speed, large field of view, long operation distance, and low price, but has low precision and is susceptible to interference from ambient light.

Stereo vision literally means to perceive 3D structures with one or two eyes, and generally refers to obtaining two or more images from different viewpoints to reconstruct a 3D structure or depth information of a target object. Depth perception visual cues can be categorized into Ocular cues and Binocular cues. Currently, stereo vision 3D imaging can be realized through monocular vision, binocular vision, multi(ocular) vision, or light-field 3D imaging (electronic compound eyes or array cameras).

The structured light imaging is an imaging technique based on a principle of 3D reconstruction, which projects specific structured light (e.g., stripes, lattices, etc.) onto a surface of a to-be-captured object, and then records deformations of the structured light on the surface of the object using a camera to derive 3D shape information of the surface of the object. The structured light imaging typically includes three main components: a projection system, a camera, and a computer processing system. The projection system usually illuminates a to-be-measured object through projecting a grating or a stripe pattern. A shape and a size of the grating or the stripe pattern can be adjusted as desired. The camera is used to record a structured light pattern on the surface of the to-be-measured object and convert the structured light pattern into a digital image. A resolution and a collection speed of the camera have a great impact on imaging precision and real-time performance. The computer processing system is used to process collected image data and reconstruct a 3D shape of the to-be-measured object based on deformation information of the structured light. The process usually includes steps such as image preprocessing, camera calibration, 3D reconstruction, and data visualization. With advantages of non-contact, high precision, and high efficiency, the structured light imaging technique is widely applied in industrial manufacturing, medical imaging, cultural relics protection, virtual reality, and other fields.

However, conventional biomedical body surface imaging systems are unable to present an internal organ structure and an internal organ distribution of the organism during body surface imaging. Obtaining the internal organ structure and the internal organ distribution requires use of Computed Tomography (CT) images, magnetic resonance images, or other images capable of presenting an internal structure of a to-be-imaged organism, which reduces an imaging efficiency.

Therefore, a deep learning-based method for generating an internal structure of an organism is urgently needed. Compared with the related art, the deep learning-based method realizes automatic imaging of the internal organ structure and the internal organ distribution of the organism during the body surface imaging.

### SUMMARY

The present disclosure solves a technical problem in the related art, and provides a deep learning-based method for generating an internal structure of an organism.

To realize the above objective, the present disclosure adopts the following technical solutions.

A deep learning-based method for generating an internal structure of an organism includes the following steps: S1, constructing and training an internal organ generation model of an imaging target; S2, obtaining a three-dimensional body surface contour image of the imaging target; S3, inputting the obtained three-dimensional body surface contour image of the imaging target into the internal organ generation model to obtain a three-dimensional internal organ distribution image; and S4, performing superimposition and merging on the three-dimensional internal organ distribution image and the three-dimensional body surface contour image, and displaying a result obtained based on the superimposition and the merging. Step S1 specifically includes the following steps: S101, obtaining an original three-dimensional body surface contour image of the imaging target and an internal structure image of the imaging target; S102, performing segmentation on the internal structure image to obtain a binary mask image of an organ; S103, obtaining an organ mask image registered with the original three-dimensional body surface contour image; S104: normalizing the original three-dimensional body surface contour image, taking the normalized three-dimensional body surface contour image as an input image of a deep learning neural network, taking the organ mask image as an output result of the deep learning neural network, and taking the three-dimensional body surface contour image and the organ mask image as a training data sample pair; and S105, training the internal organ generation model of the imaging target using the training data sample pair, where the internal organ generation model of the imaging target is based on a neural network.

Further, a training method for the internal organ generation model in step S105 includes: adopting a diffusion model for the deep learning neural network, performing a forward process by the diffusion model, and then performing an inverse diffusion process by the diffusion model. The forward process is a process of gradually adding a Gaussian noise. The forward process includes adding a random noise to the organ mask image of the imaging target. The inverse diffusion process is a process of learning a random noise component on the organ mask image of the imaging target under guidance of the three-dimensional body surface contour image, and denoising the organ mask image of the imaging target.

Further, the forward process is expressed by the following equation: $q \left({x}_{t}\left|{x}_{t - 1}\right.\right) = N \left({x}_{t};\sqrt{1 - {φ}_{t}}\left({x}_{t}, t\right);{φ}_{t}I\right) ,$ where *N* represents the three-dimensional body surface contour image, *φₜ* represents a hyperparameter, ranges from 0 to 1, and satisfies *φ*₁ < *φ*₂ < ··· < *φ_{T}*, t represents a Gaussian noise at a predetermined time point, *xₜ* represents a data sample with a Gaussian noise at the time point t, and I represents a unit matrix.

Further, the inverse diffusion process is a Gaussian distribution process and is specifically expressed by the following equation: $p \left({x}_{t - 1}\left|{x}_{t}, Q\right.\right) = N \left({x}_{t - 1};Q;{μ}_{θ}{x}_{t - 1};{\sum }_{θ} \left({x}_{t}, t\right)\right) ,$ where *N* represents the three-dimensional body surface contour image, each of *µ*_{θ} and ∑*_{θ}*(*xₜ*, *t*) represents a learning parameter, and Q represents the normalized three-dimensional body surface contour image in step S104.

Further, a method for obtaining the binary mask image of the organ in step S102 specifically includes: performing segmentation to obtain an organ contour of the imaging target in the internal structure image, and assigning a value of 1 to a region outside the organ contour and assigning a value of 0 to a region within the organ contour, to obtain a binary mask image of a target organ.

Further, obtaining the organ mask image in step S103 specifically includes: registering the binary mask image with the original three-dimensional body surface contour image to obtain a contour registration displacement field, and applying the contour registration displacement field to the binary mask image to obtain the organ mask image registered with the three-dimensional body surface contour image.

Further, a method for registering the binary mask image with the original three-dimensional body surface contour image includes: for an original three-dimensional body surface contour image and an organ mask image of a same cross section, a body surface contour curve being behind the cross section of the original three-dimensional body surface contour image, elastically registering an edge of the organ mask image with the body surface contour curve to obtain an elastic registration displacement field, and applying the elastic registration displacement field on an organ structure in the organ mask image.

Further, the method for registering the binary mask image with the original three-dimensional body surface contour image includes: performing curved surface registration on the original three-dimensional body surface contour image and a three-dimensional contour of the binary mask image to obtain an elastic registration displacement field, and applying the elastic registration displacement field on an organ structure in the organ mask image.

Further, the normalizing the original three-dimensional body surface contour image is performed through the following formula: ${Q}^{j} = \frac{{P}^{j} - {P}_{min}^{j}}{{P}_{max}^{j} - {P}_{min}^{j}} ,$ where Q^{j} represents a j-th normalized three-dimensional body surface contour image, P^{j} represents a j-th original three-dimensional body surface contour image, ${P}_{min}^{j}$ represents a minimum value in the j-th original three-dimensional body surface contour image, and ${P}_{max}^{j}$ represents a maximum value in the j-th original three-dimensional body surface contour image.

Compared with the related art, the present disclosure can provide the following advantageous effects. With a deep neural network-based internal organ generation model of the imaging target in the present disclosure, the three-dimensional internal organ distribution image of the imaging target can be obtained quickly and accurately simply through obtaining the three-dimensional body surface contour image of the imaging target and inputting the three-dimensional body surface contour image of the imaging target into the deep neural network-based internal organ generation model of the imaging target, without performing additional internal structure imaging operations, which simplifies operation steps, realizes automatic imaging, improves an imaging efficiency, and has high applicability.

### BRIEF DESCRIPTION OF THE DRAWINGS

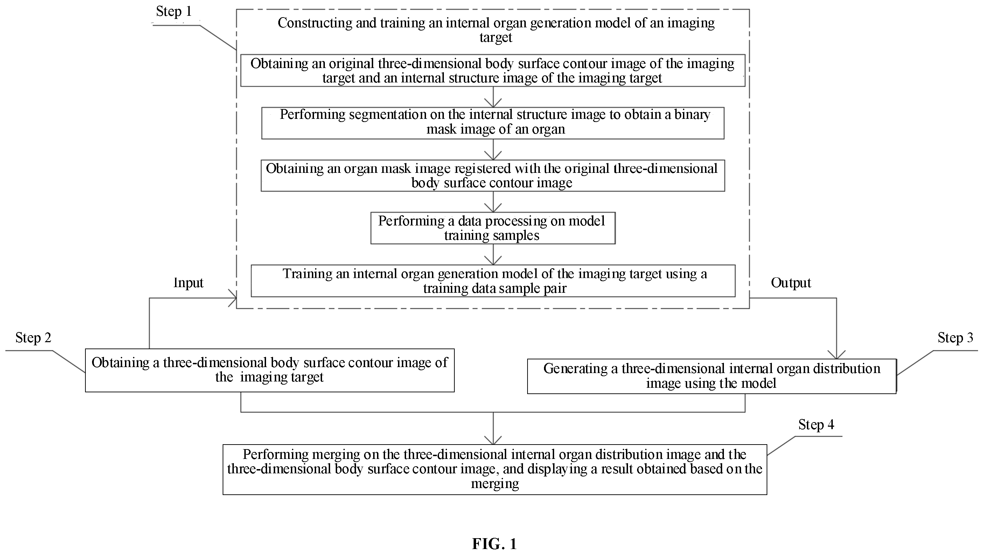

FIG. 1 is a flowchart of a method according to the present disclosure.

### DETAILED DESCRIPTION

Technical solutions of the present disclosure will be described clearly below in combination with accompanying drawings. Obviously, the embodiments described below are not all embodiments of the present disclosure. All other embodiments obtained by those skilled in the art without creative labor shall fall within the protection scope of the present disclosure.

As illustrated in FIG. 1, the present disclosure provides a deep learning-based method for generating an internal structure of an organism. The deep learning-based method includes the following steps.

In S1, an internal organ generation model of an imaging target is constructed and trained. Step S1 specifically includes the following steps.

In S101, an original three-dimensional body surface contour image of the imaging target and an internal structure image of the imaging target are obtained.

Specifically, the original three-dimensional body surface contour image of the imaging target may be obtained through optical body surface imaging or through other means such as radar. The internal structure image may be a CT image, a magnetic resonance image, or other images that can present the internal structure of the to-be-imaged organism.

In S102, segmentation is performed on the internal structure image to obtain a binary mask image of an organ. An organ contour of the imaging target is obtained by performing segmentation on the internal structure image obtained in step S101. A value of 1 is assigned to a region outside the organ contour and a value of 0 is assigned to a region within the organ contour, to obtain a binary mask image of a target organ.

In S103, an organ mask image registered with the original three-dimensional body surface contour image is obtained. The binary mask image is registered with the original three-dimensional body surface contour image obtained in step S101 to obtain a contour registration displacement field. The obtained contour registration displacement field is applied to the binary mask image to obtain the organ mask image registered with the original three-dimensional body surface contour image.

Specifically, a method for registering the binary mask image with the original three-dimensional body surface contour image includes: for an original three-dimensional body surface contour image and an organ mask image of a same cross section, a body surface contour curve being behind the cross section of the original three-dimensional body surface contour image, elastically registering an edge of the organ mask image with the body surface contour curve to obtain an elastic registration displacement field, and applying the elastic registration displacement field on an organ structure in the organ mask image. In this way, the registered organ mask image is obtained. Alternatively, curved surface registration is performed on the original three-dimensional body surface contour image and a three-dimensional contour of the binary mask image to obtain an elastic registration displacement field. The elastic registration displacement field is applied on an organ structure in the organ mask image.

In S104, data processing is performed on model training samples. The original three-dimensional body surface contour image is normalized to obtain the normalized three-dimensional body surface contour image. The original three-dimensional body surface contour image is normalized through the following formula: ${Q}^{j} = \frac{{P}^{j} - {P}_{min}^{j}}{{P}_{max}^{j} - {P}_{min}^{j}} ,$ where Q^{j} represents a j-th normalized three-dimensional body surface contour image, P^{j} represents a j-th original three-dimensional body surface contour image, ${P}_{min}^{j}$ represents a minimum value in the j-th original three-dimensional body surface contour image, and ${P}_{max}^{j}$ represents a maximum value in the j-th original three-dimensional body surface contour image.

The normalized three-dimensional body surface contour image is denoted as N. The normalized three-dimensional body surface contour image N is taken as an input image of a deep learning neural network. The registered organ mask image of the imaging target is taken as an output result. The three-dimensional body surface contour image and the organ mask image of the imaging target corresponding to the three-dimensional body surface contour image are taken as a training data sample pair.

In S105, the internal organ generation model of the imaging target is trained using the training data sample pair. A deep neural network-based internal organ generation model of the imaging target is constructed. The training data sample pairs are inputted into the deep neural network for training. The deep neural network adopts a diffusion model. The diffusion model operates by adding a random noise to the organ mask image of the imaging target, learning a random noise component on an organ mask image of the imaging target under guidance of the three-dimensional body surface contour image, and denoising the organ mask image of the imaging target, to obtain a relevant organ structure of the imaging target.

In particular, the diffusion model is derived from equilibrium thermodynamics. A Markov chain of diffusion steps is set up. A random noise is gradually added to real data. Such a process is referred to as a forward process. Then, an inverse denoising process is learned. The inverse denoising process is referred to as an inverse diffusion process. A desired data sample result is obtained from the noise. The forward process is a process of gradually adding a Gaussian noise. For example, an input data distribution is *x~q*(*x*)*.* In the forward process, the Gaussian noise is added a total of T times. Therefore, a series of data samples with the Gaussian noise are generated, the data samples are represented as *x*₁, *x*₂, *x*₃, ..., and *xₜ.* The forward process is expressed by the following equation: $q \left({x}_{t}\left|{x}_{t - 1}\right.\right) = N \left({x}_{t};\sqrt{1 - {φ}_{t}}\left({x}_{t}, t\right);{φ}_{t}I\right) ,$ where *φₜ* represents a hyperparameter, ranges from 0 to 1, and satisfies *φ*₁ < *φ*₂ < ... < *φ_{T},* t represents a Gaussian noise at a predetermined time point, and I represents a unit matrix.

The inverse diffusion process is a process of gradually recovering an original image (the organ mask image) from the Gaussian noise under the guidance of the three-dimensional body surface contour image. A small amount of Gaussian noise is added to the forward process each time. Therefore, the inverse diffusion process can also be considered as a Gaussian distribution. The entire process can be simulated using a neural network. The inverse diffusion process is expressed by the following equation: $p \left({x}_{t - 1}\left|{x}_{t}, Q\right.\right) = N \left({x}_{t - 1};Q;{μ}_{θ}{x}_{t - 1};{\sum }_{θ} \left({x}_{t}, t\right)\right) ,$ where *p*(*x*_{*t*-1}|*xₜ, Q*) represents the inverse diffusion process, *N* represents the three-dimensional body surface contour image, each of *µ_{θ}* and Σ*_{θ}*(*xₜ, t*) represents a learning parameter, and Q represents the normalized three-dimensional body surface contour image obtained in step S104.

In S2, a three-dimensional body surface contour image of the imaging target is obtained.

In S3, a three-dimensional distribution image is generated using the internal organ generation model of the imaging target. The three-dimensional body surface contour image obtained in step S2 is inputted into the internal organ generation model of the imaging target to obtain the organ mask image of the imaging target, i.e., a three-dimensional internal organ distribution image.

In S4, superimposition and merging are performed on the three-dimensional internal organ distribution image and the three-dimensional body surface contour image, and a result obtained based on the superimposition and the merging is displayed.

With the deep neural network-based internal organ generation model of the imaging target in the present disclosure, the three-dimensional internal organ distribution image of the imaging target can be obtained quickly and accurately simply through obtaining the three-dimensional body surface contour image of the imaging target and inputting the three-dimensional body surface contour image of the imaging target into the deep neural network-based internal organ generation model of the imaging target, without performing additional internal structure imaging operations, which simplifies operation steps, improves an imaging efficiency, and has high applicability.

Finally, it should be noted that the above contents are used only to explain technical solutions of the present disclosure, rather than to limit the protection scope of the present disclosure. Simple modifications or equivalent replacements can be made to the technical solutions of the present disclosure by those skilled in the art, without departing from the essence and the scope of the technical solutions of the present disclosure.

## Claims

1. A deep learning-based method for generating an internal structure of an organism, the deep learning-based method comprising the following steps:
S1, constructing and training an internal organ generation model of an imaging target;
S2, obtaining a three-dimensional body surface contour image of the imaging target;
S3, inputting the obtained three-dimensional body surface contour image of the imaging target into the internal organ generation model to obtain a three-dimensional internal organ distribution image; and
S4, performing superimposition and merging on the three-dimensional internal organ distribution image and the three-dimensional body surface contour image, and displaying a result obtained based on the superimposition and the merging,
wherein step S1 comprises the following steps:
S101, obtaining an original three-dimensional body surface contour image of the imaging target and an internal structure image of the imaging target;
S102, performing segmentation on the internal structure image to obtain a binary mask image of an organ;
S103, obtaining an organ mask image registered with the original three-dimensional body surface contour image, wherein step S103 comprises: registering the binary mask image with the original three-dimensional body surface contour image to obtain a contour registration displacement field, and applying the contour registration displacement field to the binary mask image to obtain the organ mask image registered with the three-dimensional body surface contour image, wherein:
said registering the binary mask image with the original three-dimensional body surface contour image comprises: for an original three-dimensional body surface contour image and an organ mask image of a same cross section, a body surface contour curve being behind the cross section of the original three-dimensional body surface contour image, elastically registering an edge of the organ mask image with the body surface contour curve to obtain an elastic registration displacement field, and applying the elastic registration displacement field on an organ structure in the organ mask image; or
said registering the binary mask image with the original three-dimensional body surface contour image comprises: performing curved surface registration on the original three-dimensional body surface contour image and a three-dimensional contour of the binary mask image to obtain an elastic registration displacement field, and applying the elastic registration displacement field on an organ structure in the organ mask image;
S104: normalizing the original three-dimensional body surface contour image, taking the normalized three-dimensional body surface contour image as an input image of a deep learning neural network, taking the organ mask image as an output result of the deep learning neural network, and taking the three-dimensional body surface contour image and the organ mask image as a training data sample pair; and
S105, training the internal organ generation model of the imaging target using the training data sample pair, wherein the internal organ generation model of the imaging target is based on a neural network.

2. The deep learning-based method for generating the internal structure of the organism according to claim 1, wherein said training the internal organ generation model in step S105 comprises:
adopting a diffusion model for the deep learning neural network, performing a forward process by the diffusion model, and then performing an inverse diffusion process by the diffusion model, wherein:
the forward process is a process of gradually adding a Gaussian noise, wherein the forward process comprises adding a random noise to the organ mask image of the imaging target; and
the inverse diffusion process is a process of learning a random noise component on the organ mask image of the imaging target under guidance of the three-dimensional body surface contour image, and denoising the organ mask image of the imaging target.

3. The deep learning-based method for generating the internal structure of the organism according to claim 2, wherein the forward process is expressed by the following equation: $q \left({x}_{t}\left|{x}_{t - 1}\right.\right) = N \left({x}_{t};\sqrt{1 - {φ}_{t}}\left({x}_{t}, t\right);{φ}_{t}I\right) ,$ where *N* represents the three-dimensional body surface contour image; *φₜ* represents a hyperparameter, ranges from 0 to 1, and satisfies *φ*₁ *< φ₂ < ··· < φ_{T}* ; t represents a Gaussian noise at a predetermined time point; *xₜ* represents a data sample with a Gaussian noise at the time point t; and I represents a unit matrix.

4. The deep learning-based method for generating the internal structure of the organism according to claim 2, wherein the inverse diffusion process is a Gaussian distribution process and is expressed by the following equation: $p \left({x}_{t - 1}\left|{x}_{t}, Q\right.\right) = N \left({x}_{t - 1};Q;{μ}_{θ}{x}_{t - 1};{\sum }_{θ} \left({x}_{t}, t\right)\right) ,$ where *N* represents the three-dimensional body surface contour image, each of *φ_{θ}* and Σ*_{θ}*(*xₜ, t*) represents a learning parameter, and Q represents the normalized three-dimensional body surface contour image in step S104.

5. The deep learning-based method for generating the internal structure of the organism according to claim 1, wherein said obtaining the binary mask image of the organ in step S102 comprises:
performing segmentation to obtain an organ contour of the imaging target in the internal structure image, and assigning a value of 1 to a region outside the organ contour and assigning a value of 0 to a region within the organ contour, to obtain a binary mask image of a target organ.

6. The deep learning-based method for generating the internal structure of the organism according to claim 1, wherein said normalizing the original three-dimensional body surface contour image is performed through the following formula: ${Q}^{j} = \frac{{P}^{j} - {P}_{min}^{j}}{{P}_{max}^{j} - {P}_{min}^{j}} ,$ where Q^{j} represents a j-th normalized three-dimensional body surface contour image, P^{j} represents a j-th original three-dimensional body surface contour image, ${P}_{min}^{j}$ represents a minimum value in the j-th original three-dimensional body surface contour image, and ${P}_{max}^{j}$ represents a maximum value in the j-th original three-dimensional body surface contour image.
